# EUROPÄISCHE PATENTANMELDUNG

(11) **EP 3 415 009 A1**
(43) Veröffentlichungstag der Anmeldung: **19.12.2018**
(21) Anmeldenummer: 17175539.0
(22) Anmeldetag: 12.06.2017
(51) Int. Cl.: A01N 59/00, A01N 59/16, A01N 25/34, A01P 1/00, A61L 2/18

(54) **DESINFEKTIONSTUCH**

(71) Anmelder: Wilbert Hygiene GmbH, 80807 München (DE)
(72) Erfinder: Wilbert, Thomas, 80805 München (DE)
(74) Vertreter: 2s-ip Schramm Schneider Bertagnoll Patent- und Rechtsanwälte Part mbB

(57) **Zusammenfassung**

Bereitgestellt wird durch die Erfindung ein Desinfektionssystem für die Flächendesinfektion, welches gebildet wird aus
- einem Trägermaterial und
- einer Desinfektionslösung, mit der das Trägermaterial getränkt ist,
wobei die Desinfektionslösung
a) 0,5 bis 10 Gew.-% Wasserstoffperoxid,
b) 0,025 bis 0,2 Gew.-% Phosphorsäure,
c) 0,0005 bis 0,2 Gew.-% Silber, und
d) auf 100 Gew.-% ergänzte Anteile Wasser
aufweist, jeweils bezogen auf die Gesamtzusammensetzung der Desinfektionslösung, und wobei die Desinfektionslösung bei 20°C auf einen pH-Wert von zwischen 1 und 5 eingestellt ist. Erfindungsgemäß bereitgestellt wird ferner die Verwendung einer solchen Desinfektionslösung zum Tränken eines Trägermaterials, das ausgewählt ist aus der Gruppe umfassend Vlies, textiles Gewebe, Tuch aus Baumwolle, Mikrofaser, Cellulose oder modifizierter Cellulose, Verbundstoff, und PET-Vlies.

## Beschreibung

### Gegenstand der Erfindung

Die Erfindung betrifft ein Desinfektionssystem für die Flächendesinfektion, das aus einem Trägermaterial und einer Desinfektionslösung gebildet wird, wobei das Trägermaterial mit der Desinfektionslösung getränkt ist. Die Erfindung betrifft ferner die Verwendung einer Desinfektionslösung zum Tränken eines Trägermaterials.

### Hintergrund der Erfindung

Mit einer Desinfektionslösung getränkte Desinfektionstücher sind aus dem Stand der Technik bekannt, etwa mit Ammoniumverbindungen oder verschiedenen Alkoholen getränkte Tücher. Solche mit einer Desinfektionslösung vorgetränkte Desinfektionstücher werden meist gebrauchsfertig angeboten, etwa in Form von Spenderboxen, aus denen die feuchten Desinfektionstücher einzeln entnommen werden können.

Die aus dem Stand der Technik bekannten mit einer Desinfektionslösung vorgetränkten Desinfektionstücher haben allerdings den Nachteil, dass sie meist sehr unangenehm riechen. Um den unangenehmen Geruch zu beseitigen oder zumindest teilweise zu überdecken werden der Desinfektionslösung meist Duftstoffe zugegeben. Es besteht allerdings der Wunsch auf solche Duftstoffe möglichst weitgehend zu verzichten, da diese beim Verwender zu Hautirritationen führen können, wenn nicht geeignete Schutzmaßnahmen ergriffen werden. Zudem führt der Einsatz von Duftstoffen zu einer Verteuerung des fertigen Produktes.

Ein weiteres Problem bei vielen aus dem Stand der Technik bekannten gebrauchsfertig getränkten Desinfektionstüchern liegt darin, dass diese relativ schnell austrocknen und so ihre desinfizierende Wirkung ebenfalls relativ schnell verlieren. Ebenfalls nachteilig ist es, dass nach dem ersten Öffnen der Spenderbox die desinfizierende Wirkung ebenfalls relativ schnell nachlässt, da die Spenderbox meist nicht absolut dicht wiederverschlossen werden kann und daher Teile der Desinfektionslösung - wenn auch langsam - als Gas aus der Spenderbox entweichen können.

### Aufgabe der Erfindung

Aufgabe der vorliegenden Erfindung ist es daher, Lösungen bereitzustellen, die die aus dem Stand der Technik bekannten Nachteile zumindest teilweise vermeiden und die es ermöglichen ein vorgetränktes Desinfektionstuch bereitzustellen, das einerseits einfach und kostengünstig herstellbar ist und das sich andererseits durch eine lange Haltbarkeit auszeichnet ohne dass das Desinfektionstuch unangenehm riecht.

### Erfindungsgemäße Lösung

Diese Aufgabe wird erfindungsgemäß mit einem Desinfektionssystem für die Flächendesinfektion und mit der Verwendung einer Desinfektionslösung zum Tränken eines Trägermaterials nach den unabhängigen Ansprüchen gelöst. Vorteilhafte Ausgestaltungen und Weiterbildungen der Erfindung sind in den jeweiligen abhängigen Ansprüchen angegeben.

Bereit gestellt wird demnach ein Desinfektionssystem für die Flächendesinfektion, welches gebildet wird aus
- einem Trägermaterial und
- einer Desinfektionslösung, mit der das Trägermaterial getränkt ist,
wobei die Desinfektionslösung
a) 0,5 bis 10 Gew.-% Wasserstoffperoxid,
b) 0,025 bis 0,2 Gew.-% Phosphorsäure,
c) 0,0005 bis 0,2 Gew.-% Silber, und
d) auf 100 Gew.-% ergänzte Anteile Wasser
aufweist, jeweils bezogen auf die Gesamtzusammensetzung der Desinfektionslösung, und wobei die Desinfektionslösung bei 20°C auf einen pH-Wert von zwischen 1 und 5 eingestellt ist.

Durch die Beigabe von Silber zur Desinfektionslösung, was beispielsweise in Form eines Silbersalzes erfolgen kann, wird in vorteilhafter Weise die Langzeitwirkung der Desinfektionslösung verbessert, sodass gereinigte Flächen länger steril bleiben. Ferner hat sich überraschender Weise herausgestellt, dass das beigegebene Silber den unangenehmen Geruch des Wasserstoffperoxids nahezu vollständig beseitigt, sodass keine oder allenfalls sehr geringe Mengen eines Duftstoffes hinzugefügt werden müssen, was sich in geringeren Produktionskosten auswirkt.

Es hat sich ebenfalls herausgestellt, dass sich durch die Beigabe des Silbers die Haltbarkeit und insbesondere die desinfizierende Wirksamkeit der Desinfektionslösung erheblich verlängern lassen. Zudem haben Versuche gezeigt, dass die beigefügte Phosphorsäure dafür verantwortlich ist, dass die Neigung des Wasserstoffperoxids in Wasser und Sauerstoff zu zerfallen erheblich reduziert werden kann, was wiederum zu einer deutlich längeren Wirksamkeit der Desinfektionslösung führt.

In einer vorteilhaften Ausgestaltung der Erfindung weist die Desinfektionslösung
a) 1 bis 5 Gew.-% Wasserstoffperoxid,
b) 0,05 bis 0,1 Gew.-% Phosphorsäure, und
c) 0,001 bis 0,1 Gew.-% Silber
auf, jeweils bezogen auf die Gesamtzusammensetzung der Desinfektionslösung.

Vorteilhaft ist es zudem, wenn die Desinfektionslösung bei 20°C auf einen pH-Wert von zwischen 2 und 4 eingestellt ist.

Als Wasser kann destilliertes oder vollentsalztes Wasser verwendet werden.

Das Trägermaterial kann ausgewählt sein aus der Gruppe umfassend Vlies, textiles Gewebe, Tuch aus Baumwolle, Mikrofaser, Cellulose oder modifizierter Cellulose, Verbundstoff, und PET-Vlies.

In einer vorteilhaften Ausgestaltung der Erfindung kann das Trägermaterial mit ein bis zehn Gramm Desinfektionslösung pro Gramm Trägermaterial getränkt sein.

In einer weiteren Ausgestaltung der Erfindung kann die Desinfektionslösung Duftstoffe enthalten.

Bereit gestellt wird durch die Erfindung ferner die Verwendung einer Desinfektionslösung, die
a) 0,5 bis 10 Gew.-% Wasserstoffperoxid,
b) 0,025 bis 0,2 Gew.-% Phosphorsäure,
c) 0,0005 bis 0,2 Gew.-% Silber, und
d) auf 100 Gew.-% ergänzte Anteile Wasser
aufweist, jeweils bezogen auf die Gesamtzusammensetzung der Desinfektionslösung, zum Tränken eines Trägermaterials.

Vorteilhafterweise ist hier der pH-Wert der Desinfektionslösung bei 20°C zwischen 1 und 5 eingestellt

Bei der Verwendung der Desinfektionslösung kann das Trägermaterial ausgewählt sein aus der Gruppe umfassend Vlies, textiles Gewebe, Tuch aus Baumwolle, Mikrofaser, Cellulose oder modifizierter Cellulose, Verbundstoff, und PET-Vlies.

Vorteilhafterweise weist die Desinfektionslösung bei der erfindungsgemäßen Verwendung
a) 1 bis 5 Gew.-% Wasserstoffperoxid,
b) 0,05 bis 0,1 Gew.-% Phosphorsäure, und
c) 0,001 bis 0,1 Gew.-% Silber
auf, jeweils bezogen auf die Gesamtzusammensetzung der Desinfektionslösung.

Weiter vorteilhaft ist es, wenn die Desinfektionslösung bei 20°C auf einen pH-Wert von zwischen 2 und 4 eingestellt ist.

Bei der Verwendung der Desinfektionslösung kann zum Tränken des Trägermaterials 1 bis 10 Gramm Desinfektionslösung pro Gramm Trägermaterial verwendet werden.

Das erfindungsgemäße Desinfektionssystem eignet sich besonders gut zur Aufbewahrung in Behältern oder Verpackungen, bei denen nach dem erstmaligen Öffnen nicht garantiert werden kann, dass sie wieder vollständig luftdicht wiederverschließbar sind. Denn es hat sich gezeigt, dass sich eine Verringerung der desinfizierenden Wirksamkeit der Desinfektionslösung in nicht vollständig luftdicht wiederverschließbaren Behältern oder Verpackungen erst nach Wochen oder gar Monaten nachweisen lässt.

### Detaillierte Beschreibung der Erfindung

Das erfindungsgemäße Desinfektionssystem eignet sich insbesondere für die Flächendesinfektion in Krankenhäusern, Arztpraxen, Zahnarztpraxen und/oder anderen klinischen / medizinischen Einrichtungen.

Für die Herstellung des erfindungsgemäßen Desinfektionssystems wurde zunächst die Desinfektionslösung hergestellt, mit der anschließend das Trägermaterial getränkt wurde.

Die Desinfektionslösung wurde hergestellt, indem Wasserstoffperoxid, Phosphorsäure, Silber und destilliertes Wasser unter Rühren vermischt und homogenisiert wurden, wobei für einen Kilogramm Desinfektionslösung etwa zwischen 5 g und 100 g Wasserstoffperoxid, etwa zwischen 0,25 g und 2 g Phosphorsäure, etwa zwischen 0,005 g und 2 g Silber verwendet wurden.

In Abhängigkeit von den verwendeten Mengen des Wasserstoffperoxids und des Silbers wurde soviel Phosphorsäure zugegeben, bis der pH-Wert der Desinfektionslösung bei Raumtemperatur (ca. 20°C) etwa zwischen 1 und 5, vorzugsweise jedoch zwischen 2 und 4 betrug.

Das Silber kann in Form eines Silbersalzes, etwa Silbernitrat zugegeben werden.

Nach der Herstellung der Desinfektionslösung wurde das Trägermaterial mit dieser Desinfektionslösung getränkt. Als Trägermaterial wurden Vliese, textile Gewebe, Tücher aus Baumwolle oder Viskose, Mikrofasertücher, Tücher aus Cellulose oder modifizierter Cellulose, Verbundstoffe und Polyestervliesstoffe, etwa PET-Vliese verwendet.

Als besonders geeignet haben sich Trägermaterialen erwiesen, die die Wirkstoffe der Desinfektionslösung möglichst wenig adsorbieren, etwa Vliesstoffe auf Polymerbasis, z.B. Polyestervliesstoffe wie zum Beispiel PET-Vliese. Dadurch kann die Menge, mit der das Trägermaterial getränkt werden muss, zum Teil erheblich reduziert werden, ohne dass sich Einbußen bei der Desinfektionsleistung ergeben.

In Versuchen wurden die Trägermaterialien je nach Material mit etwa zwischen 1 g und 10 g Desinfektionslösung pro Gramm Trägermaterial getränkt. Bei dem PET-Vlies hat sich gezeigt, dass etwa zwischen 1 g und 2g Desinfektionslösung pro Gramm Trägermaterial ausreichend waren, um die gewünschte Desinfektionsleistung zu erreichen. Bei den anderen Materialien musste etwas mehr Desinfektionslösung pro Gramm Trägermaterial verwendet werden.

In Versuchen wurden folgende Desinfektionslösungen DL 1, DL 2 und DL 3 hergestellt und damit jeweils ein PET-Vlies getränkt (Angaben in Gew.-%).

| Bestandteil | DL 1 | DL 2 | DL 2 |
|---|---|---|---|
| Wasserstoffperoxid | 1,5 | 5,0 | 1,5 |
| Phosphorsäure | 0,075 | 0,1 | 0,075 |
| Silber | 0,1 | 0,0075 | 0,0025 |
| destilliertes Wasser | Rest | Rest | Rest |
| Menge pro Gramm Trägermaterial | 2 g | 1 g | 2 g |

Bei der Desinfektionslösung DL 1 konnte eine sehr gute biozide Wirksamkeit gegen Bakterien, Bio filme, Hefen und Viren, insbesondere Influenzaviren beobachtet werden.

Bei der Desinfektionslösung DL 2 konnte eine sehr gute biozide Wirksamkeit gegen bakterielle Endosporen, Bakterien, Hefen, Pilze und Viren beobachtet werden. Zudem konnte eine sehr gute Hemmung der Wiederverkeimung beobachtet werden - ein Wiederverkeimung erfolgte in Tests regelmäßig erst nach mindestens 18 Stunden, was auf den im Vergleich zu den Desinfektionslösungen DL 1 und DL 3 höheren Wasserstoffperoxid-Anteil zurückzuführen war.

Die Desinfektionslösung DL 3 zeigte eine gute Wirksamkeit gegen Bakterien, Hefen und Viren. Bei den Desinfektionslösungen DL 1 und DL 3 konnte eine Wiederverkeimung erst nach frühestens 12 Stunden festgestellt werden.

Die erfindungsgemäß getränkten Trägermaterialien, d.h. erfindungsgemäß getränkten Tücher eignen sich daher besonders gut zur Flächendesinfektion in klinischen Umgebungen und Arztpraxen sowie zur Reinigung von medizinischen Geräten und Medizinprodukten.

Die erfindungsgemäß getränkten Trägermaterialien (bei den Versuchen wurden Tücher verwendet) wurden stapelweise in einer luftdichten Verpackung gelagert, wobei die Tücher auch lösbar miteinander verbunden sein können. Hier hat sich überraschender Weise gezeigt, dass auch nach mehreren Monaten nahezu keine Reduzierung der bioziden Wirksamkeit eingetreten war. Nach mehrmaligen Öffnen und (nicht vollständig luftdichten) Wiederverschließen der Verpackung (in Versuchen wurde die Verpackung zweimal täglich geöffnet und jeweils ein Tuch entnommen) war eine leichte Reduzierung der bioziden Wirksamkeit erst nach mehreren Wochen feststellbar. Wurde als Verpackung ein Eimer verwendet, der sich weitgehend luftdicht wiederverschließen ließ, war eine Reduzierung der bioziden Wirksamkeit deutlich später feststellbar.

Erfindungsgemäß getränkte Tücher können beispielsweise im Flowpack mit verschiedenen Tüchergrößen und verschiedener Anzahl von Tüchern bereitgestellt werden. In einem Flowpack können beispielsweise 10, 20, 50 oder 100 getränkte Tücher aufgenommen werden. Größere Flowpacks sind aber möglich, insbesondere deshalb, da die Verringerung der bioziden Wirkung der getränkten Tücher erst nach Wochen oder gar Monaten nachweisbar ist. Damit eignen sich die erfindungsgemäß getränkten Tücher besonders gut für die Verwendung in Behältnissen, die nach dem erstmaligen Öffnen nicht mehr vollständig luftdicht wiederverschließbar sind.

Alternativ kann auch eine mit der Desinfektionslösung getränkte Tuchrolle in einen Spendereimer gegeben werden, aus dem die benötigte Länge des Tuches entnommen werden kann und von der Tuchrolle abgerissen werden kann. Möglich wäre auch das Tränken einer Tuchrolle mit der Desinfektionslösung nachdem die Tuchrolle in den Spendereimer gegeben wurde. Eine getränkte Tuchrolle kann beispielsweise 40, 100, 120 oder 200 abtrennbare Tücher aufweisen. Größere Rollen sind aber auch möglich.

Im Ergebnis konnte überraschender Weise festgestellt werden, dass bei der Verwendung der vorstehend beschriebenen Desinfektionslösung zum Tränken eines Trägermaterials und bei einem Trägermaterial, das mit der vorstehend beschriebenen Desinfektionslösung getränkt wurde, auf einfache und kostengünstige Weise ein Desinfektionstuch bzw. Desinfektionssystem herstellbar ist, das sich zudem durch eine lange Haltbarkeit und durch eine deutlich verbesserte Langzeitwirkung auszeichnet, wobei gleichzeitig die Menge an Desinfektionslösung pro Gramm Trägermaterial deutlich gesenkt werden konnte, ohne dass Einbußen in der bioziden Wirksamkeit hingenommen werden müssen. Damit muss auch deutlich weniger Desinfektionslösung der Entsorgung zugeführt werden, wodurch auch die Umwelt entlastet wird.

## Patentansprüche

1. Desinfektionssystem für die Flächendesinfektion, welches gebildet wird aus
- einem Trägermaterial und
- einer Desinfektionslösung, mit der das Trägermaterial getränkt ist,
wobei die Desinfektionslösung
a) 0,5 bis 10 Gew.-% Wasserstoffperoxid,
b) 0,025 bis 0,2 Gew.-% Phosphorsäure,
c) 0,0005 bis 0,2 Gew.-% Silber, und
d) auf 100 Gew.-% ergänzte Anteile Wasser
aufweist, jeweils bezogen auf die Gesamtzusammensetzung der Desinfektionslösung, und wobei die Desinfektionslösung bei 20°C auf einen pH-Wert von zwischen 1 und 5 eingestellt ist.

2. Desinfektionssystem nach dem vorhergehenden Anspruch, wobei die Desinfektionslösung
a) 1 bis 5 Gew.-% Wasserstoffperoxid,
b) 0,05 bis 0,1 Gew.-% Phosphorsäure, und
c) 0,001 bis 0,1 Gew.-% Silber
aufweist, jeweils bezogen auf die Gesamtzusammensetzung der Desinfektionslösung, und/oder wobei die Desinfektionslösung bei 20°C auf einen pH-Wert von zwischen 2 und 4 eingestellt ist.

3. Desinfektionssystem nach einem der vorhergehenden Ansprüche, wobei das Wasser destilliertes oder vollentsalztes Wasser ist.

4. Desinfektionssystem nach einem der vorhergehenden Ansprüche, wobei das Trägermaterial ausgewählt ist aus der Gruppe umfassend Vlies, textiles Gewebe, Tuch aus Baumwolle, Mikrofaser, Cellulose oder modifizierter Cellulose, Verbundstoff, und PET-Vlies.

5. Desinfektionssystem nach einem der vorhergehenden Ansprüche, wobei das Trägermaterial mit 1 bis 35 Gramm Desinfektionslösung pro Gramm Trägermaterial, insbesondere mit 1 bis 10 Gramm Desinfektionslösung pro Gramm Trägermaterial getränkt ist.

6. Desinfektionssystem nach einem der vorhergehenden Ansprüche, wobei die Desinfektionslösung Duftstoffe enthält.

7. Verwendung einer Desinfektionslösung aufweisend
a) 0,5 bis 10 Gew.-% Wasserstoffperoxid,
b) 0,025 bis 0,2 Gew.-% Phosphorsäure,
c) 0,0005 bis 0,2 Gew.-% Silber, und
d) auf 100 Gew.-% ergänzte Anteile Wasser,
jeweils bezogen auf die Gesamtzusammensetzung der Desinfektionslösung, und die bei 20°C auf einen pH-Wert von zwischen 1 und 5 eingestellt ist, zum Tränken eines Trägermaterials, das ausgewählt ist aus der Gruppe umfassend Vlies, textiles Gewebe, Tuch aus Baumwolle, Mikrofaser, Cellulose oder modifizierter Cellulose, Verbundstoff, und PET-Vlies.

8. Verwendung nach dem vorhergehenden Anspruch, wobei die Desinfektionslösung
a) 1 bis 5 Gew.-% Wasserstoffperoxid,
b) 0,05 bis 0,1 Gew.-% Phosphorsäure, und
c) 0,001 bis 0,1 Gew.-% Silber
aufweist, jeweils bezogen auf die Gesamtzusammensetzung der Desinfektionslösung, und/oder wobei die Desinfektionslösung bei 20°C auf einen pH-Wert von zwischen 2 und 4 eingestellt ist.

9. Verwendung nach einem der vorhergehenden Ansprüche, wobei zum Tränken des Trägermaterials 1 bis 10 Gramm Desinfektionslösung pro Gramm Trägermaterial verwendet wird.
